# EUROPEAN PATENT APPLICATION

(11) **EP 3 047 859 A1**
(43) Date of publication of application: **27.07.2016**
(21) Application number: 14845238.6
(22) Date of filing: 19.09.2014
(51) Int. Cl.: A61L 27/00, A61K 35/12, A61K 35/39

(54) **DEVICE AND METHOD FOR IMMUNOSUPPRESSANT-FREE TRANSPLANTATION, AND USAGE THEREOF**

(30) Priority: 20.09.2013 JP 2013195220
(71) Applicant: Kyoto University, Kyoto-shi, Kyoto 606-8501 (JP); ARKRAY, Inc., Minami-ku Kyoto-shi Kyoto 601-8045 (JP)
(72) Inventor: IWATA, Hiroo, Kyoto-shi Kyoto 606-8507 (JP); LUAN, Nguyen Minh, Kyoto-shi Kyoto 606-8507 (JP)
(74) Representative: Jones, Elizabeth Louise
(86) International application number: PCT/JP2014/074973
(87) International publication number: WO 2015/041357

(57) **Abstract**

The present invention provides a device to be implanted subcutaneously for forming a transplantation space where rejection does not occur, wherein the device comprises a biocompatible structure containing an angiogenesis inducing factor.

## Description

### TECHNICAL FIELD

The present invention relates to a device for an immunosuppressant-free transplantation, and specifically to a device which allows for transplantation of allogeneic cells or allogeneic micro-tissue (hereinafter, referred to as a transplantation material) without requiring the administration of an immunosuppressant. The present invention also relates to a method for forming a subcutaneous space within which immunological rejection is suppressed. The present invention also relates to a method for transplanting a transplantation material under immunosuppressant-free conditions. The present invention also relates to a use of a device for allowing the survival of a transplantation material, which may be rejected immunologically if particular measures are not taken to prevent the rejection, in subcutaneous tissue under immunosuppressant-free conditions.

As used herein, as the transplantation material, islet of Langerhans grafts (islet grafts) having an ability to produce insulin may be simply referred to as pancreatic islets.

### BACKGROUND ART

Currently, subcutaneous injections of insulin are performed for the treatment of insulin-dependent diabetes. In a long-term perspective, however, there are cases where vascular complications occur due to insufficient control of the blood glucose level, for example, loss of sight due to diabetic retinopathy, kidney failure due to diabetic nephropathy, necessity for leg amputation due to impaired blood circulation, and the like. Particularly, the most frequent primary disease, in patients suffering from kidney failure due to diabetic nephropathy who eventually require dialysis treatment, is diabetes. Medical cost of over one point seven trillion yen is required for the dialysis treatment, imposing a large burden on the medical insurance system. In a short term perspective, on the other hand, there are patients who suffer from hypoglycemia during sleep due to unstable blood glucose level, resulting in death. In view of the above, studies have been carried out for a long time, in which pancreatic islets separated from a pancreas provided by a deceased donor are transplanted to a diabetes patient. In recent years, this procedure has been clinically applied to approximately two to three hundred people per year around the world. In a clinical application of pancreatic islet transplantation, the pancreatic islets are transplanted into the liver through the portal vein, and an immunosuppressant is administered after the transplantation in order to prevent rejection. However, the procedure for transplanting pancreatic islets into the liver is rather invasive, and in addition, it is difficult to remove the transplanted pancreatic islets, in cases where a problem occurs in the transplanted site after the transplantation. Further, the occurrence of complications resulting from the administration of an immunosuppressant, which is carried out in order to prevent rejection, has been pointed out, for example, being susceptible to infections, having an increased risk of developing a cancer, and the like. In addition, since immunosuppressants are expensive as compared to insulin, it leads to an increased financial burden of the patients after the operation.

Therefore, a technique for allowing transplantation of pancreatic islets to a subcutaneous site has been demanded, which can be carried out using a minimally invasive procedure, and which allows for an easy removal the transplanted pancreatic islets in case of a problem. Further, as transplantation methods which do not require the administration of an immunosuppressant, various techniques have been attempted, such as the use of bioartificial pancreas, in which pancreatic islets are transplanted to a recipient after encapsulating the pancreatic islets with a semipermeable membrane to isolate them from the recipient's immune system; the use of ultraviolet light irradiation or low temperature culture in order to eliminate dendritic cells in pancreatic islets, which cells induce an immune response; and the like. However, these are associated with technical obstacles, and problems in reproducibility. Accordingly, a method for pancreatic islet transplantation which is free of the above mentioned problems is strongly demanded.

Patent Document 1 discloses a technique for inducing the formation of subcutaneous vascular bed formed from blood vessels having a small diameter, in order to transplant pancreatic islets or other cells subcutaneously. However, Patent Document 1 discusses syngeneic pancreatic islet transplantation, in which the problem of rejection need not be addressed, and it is totally silent about the rejection which occurs in allogeneic pancreatic islet transplantation. It should be noted that, "allogeneic" means that "belonging to the same species, but having a different genetic composition".

### PRIOR ART DOCUMENTS

### PATENT DOCUMENTS

Patent Document 1: JP 2000-178180 A

### SUMMARY OF THE INVENTION

### PROBLEMS TO BE SOLVED BY THE INVENTION

An object of the present invention is to provide a technique which allows for subcutaneous transplantation of cells, tissue, or the like, without requiring the administration of an immunosuppressant.

### MEANS FOR SOLVING THE PROBLEMS

The present invention provides a device, methods and a use having the following constitutions.
Item 1. A device to be implanted subcutaneously for forming a transplantation space where rejection does not occur, wherein the device comprises a biocompatible structure containing an angiogenesis inducing factor.
Item 2. The device according to item 1, wherein a material to be transplanted in the transplantation space is allogeneic cells or allogeneic micro-tissue.
Item 3. The device according to item 2, wherein the material to be transplanted in the transplantation space is pancreatic islets.
Item 4. The device according to item 2, wherein the material to be transplanted in the transplantation space is cells which have been induced to differentiate from somatic stem cells, embryonic stem cells or induced pluripotent stem cells.
Item 5. The device according to item 4, wherein the cells which have been induced to differentiate are insulin-secreting cells.
Item 6. The device according to any one of claims 1 to 5, wherein the angiogenesis inducing factor is at least one selected from the group consisting of fibroblast growth factor, vascular endothelial growth factor, hepatocyte growth factor, platelet-derived growth factor, placental growth factor and epidermal growth factor.
Item 7. The device according to any one of claims 1 to 6, wherein the biocompatible structure is selected from the group consisting of a hydrogel, a sponge and a porous medium.
Item 8. The device according to any one of claims 1 to 7, further comprising heparin or heparan sulfate.
Item 9. The device according to item 2, wherein the material to be transplanted in the transplantation space is allogeneic cells or allogeneic micro-tissue.
Item 10. The device according to any one of claims 1 to 9, wherein the device comprises the angiogenesis inducing factor in an amount capable of inducing angiogenesis of 2 to 50 mg/g tissue in terms of the amount of hemoglobin.
Item 11. A method for forming a subcutaneous space where immunological rejection is suppressed under immunosuppressant-free conditions, in a mammal, the method comprising the steps of:
   implanting the device according to any one of claims 1 to 10 subcutaneously in the mammal to allow the formation of small-diameter blood vessels around the device; and
   removing the device.
Item 12. A method for transplanting a transplantation material in a mammal under immunosuppressant-free conditions, the method comprising the steps of:
   implanting the device according to any one of claims 1 to 10 subcutaneously in the mammal to allow the formation of small-diameter blood vessels around the device;
   removing the device to form a space where immunological rejection is suppressed under immunosuppressant-free conditions; and
   transplanting in the space the transplantation material selected from the group consisting of allogeneic cells and allogeneic micro-tissues.
Item 13. The method according to item 12, wherein the transplantation material is allogeneic cells or allogeneic micro-tissue.
Item 14. A use of a device for allowing the survival of a transplantation material which has a possibility of being rejected immunologically, in subcutaneous tissue under immunosuppressant-free conditions,
   wherein the device comprises a biocompatible structure containing an angiogenesis inducing factor, and
   wherein the device is implanted in the subcutaneous tissue to allow the formation of blood vessels around the device, then the device is removed from the subcutaneous tissue to form a transplantation space where rejection does not occur, and the transplantation material which has a possibility of being rejected immunologically is transplanted in the transplantation space, to allow the survival of the transplantation material in the subcutaneous tissue under immunosuppressant-free conditions.
Item 15. The use according to item 14, wherein the material to be transplanted in the transplantation space is allogeneic cells or allogeneic micro-tissue.
Item 16. A method for treating a disease, comprising the steps of:
   implanting the device according to any one of claims 1 to 10 subcutaneously in a mammal to allow the formation of small-diameter blood vessels around the device;
   removing the device to form a space where immunological rejection is suppressed under immunosuppressant-free conditions; and
   transplanting a transplantation material for treating the disease in the space; wherein the transplantation material is selected from the group consisting of allogeneic cells and allogeneic micro-tissues.
Item 17. The method for treating a disease according to item 16, wherein the disease is diabetes, and the transplantation material is pancreatic islets.

### EFFECT OF THE INVENTION

The transplantation of pancreatic islets is clinically carried out for treating insulin dependent diabetes. However, two problems remain to be solved, that the islet transplantation is still highly invasive, and that it requires the administration of an immunosuppressant after the transplantation. The present invention relates to a device for forming a subcutaneous space where a transplantation material such as pancreatic islets can be transplanted without requiring the administration of an immunosuppressant.

When a biocompatible structure, which is formed in the shape of a plate, a sheet, a tube, a rod, or the like, and carrying a factor capable of promoting the angiogenesis of blood vessels (angiogenesis inducing factor), such as fibroblast growth factor or vascular endothelial growth factor, and heparin as required, is implanted subcutaneously in a rat with diabetes, the formation of a large number of microvessels is induced in the tissue surrounding the implanted biocompatible structure, about five to 15 days after the implantation. Surprisingly, when pancreatic islets were transplanted into the space formed by removing the hydrogel, the pancreatic islets survived for a long period of time without being rejected, even without the administration of an immunosuppressant after the transplantation. In addition, the transplanted pancreatic islets were capable of secreting insulin, thereby maintaining the blood glucose level of the rat within a normal range for a long period of time.

It is also possible to allow a transplantation material other than pancreatic islets to survive in a living body without being rejected.

### BRIEF DESCRIPTION OF THE DRAWINGS

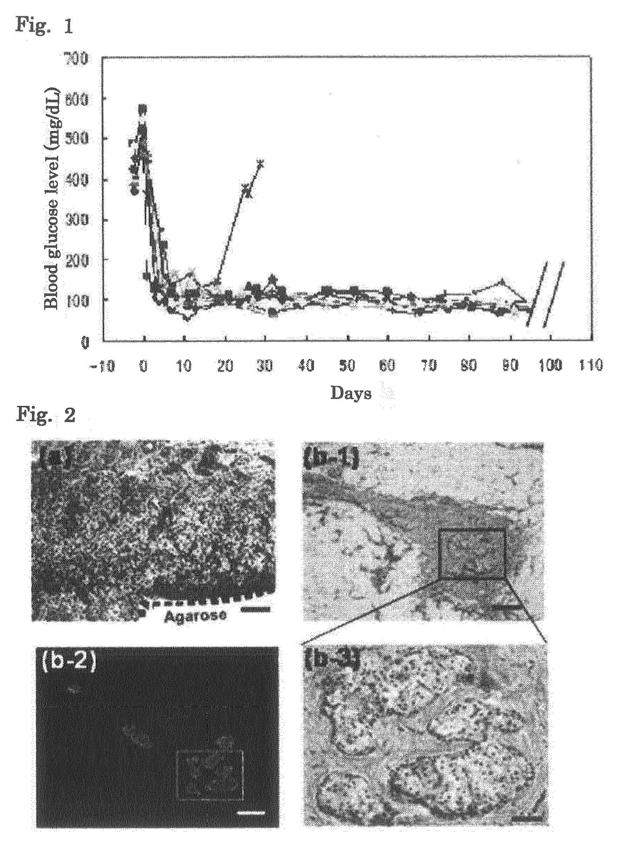

FIG. 1 shows the changes in the blood glucose levels of diabetic ACI rats, after the transplantation of pancreatic islets from F344 rats to dorsal subcutaneous sites of the ACI rats. It can be seen that the blood glucose levels were decreased due to the transplantation of allogeneic pancreatic islets, demonstrating the survival of the transplanted pancreatic islets in a state capable of secreting insulin.
FIG. 2 shows histological images of tissue sections. FIG. 2 (a) is a histological image of subcutaneous tissue in which the formation of microvessels is induced. FIG. 2 (b-1) is a haematoxylin & eosin stained image of a tissue section from the pancreatic islet transplantation site, on the day 94 after the transplantation;
FIG. 2 (b-2) is a stained image of a tissue section from an approximately the same site as the tissue shown in (b-1), immunostained for insulin; and FIG. 2 (b-3) is an enlarged view of the image shown in (b-1), showing the portion of the image in which the transplanted pancreatic islets are present. It is shown that the transplanted pancreatic islets are retaining their shapes, and that they are in a state capable of secreting insulin.

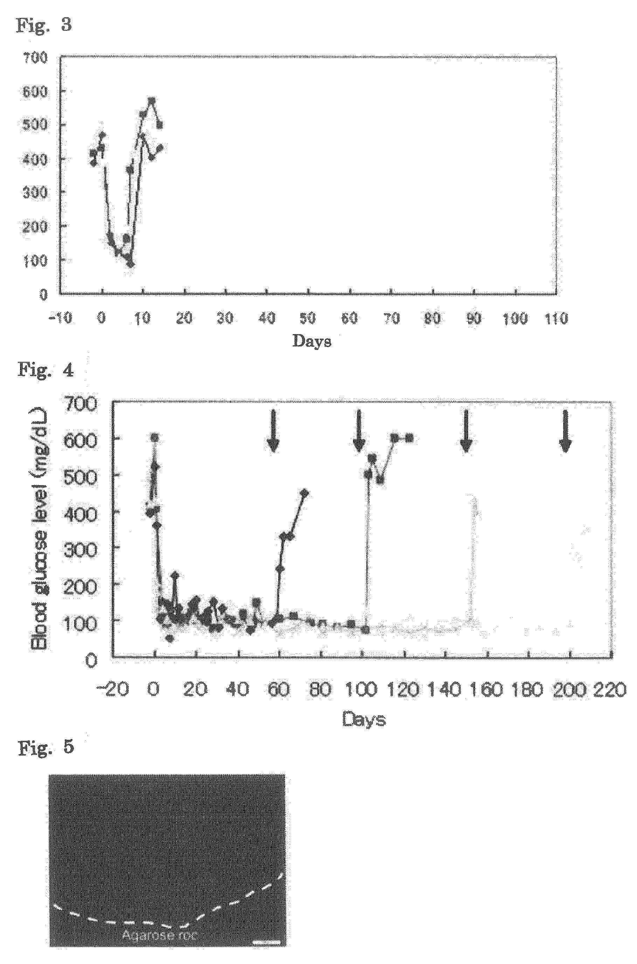

FIG. 3 shows the changes in the blood glucose levels of diabetic ACI rats, after the transplantation of pancreatic islets from the F344 rats into the livers of the ACI rats. Since the blood glucose levels were decreased once, and then increased immediately afterwards, it can be seen that the transplanted pancreatic islets were rejected.
FIG. 4 shows the changes in the blood glucose levels of diabetic ACI rats, after the transplantation of pancreatic islets from Leis rats to the ACI rats. On the days indicated with arrows in the FIG. 4, the transplanted pancreatic islets were removed along with skin. After the removal of the pancreatic islets, the blood glucose levels rapidly increased, suggesting that the decrease in the blood glucose levels was resulting from insulin secreted by the transplanted pancreatic islets.
FIG. 5 shows an image of a subcutaneous site at which angiogenesis was induced, following in vivo perfusion with FITC-lectin. A blood vascular system was identified as lectin positive blood vessels stained with green-fluorescent dye. A scale bar of 100 µm is used.

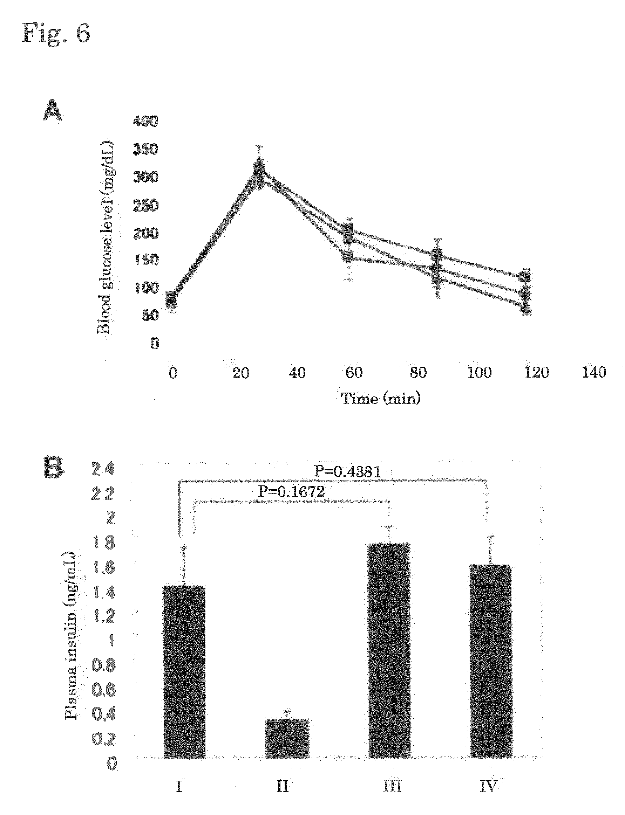

FIG. 6 (A) shows the results of an intraperitoneal glucose tolerance test in:
   normal ACI rats (triangle, n = 3); and STZ-ACI rats each transplanted with 3,000 pieces of F344 pancreatic islets (square, n = 3) or 3,000 pieces of Lewis pancreatic islets (circle, n = 3). The intraperitoneal glucose tolerance test was carried out for all the recipients, over a period of from one to three months after the transplantation. FIG. 6 (B) shows the plasma insulin levels in: non-diabetic ACI rats (I), diabetic ACI rats (II), and STZ-ACI rats each transplanted with 3,000 pieces of Lewis pancreatic islets (III) or 3,000 pieces of F344 pancreatic islets (IV) in the angiogenesis-induced subcutaneous spaces. Three recipients of allogeneic pancreatic islets in each of the groups were examined for a period of time from one to three months after the transplantation. There was no significant difference between the three groups.

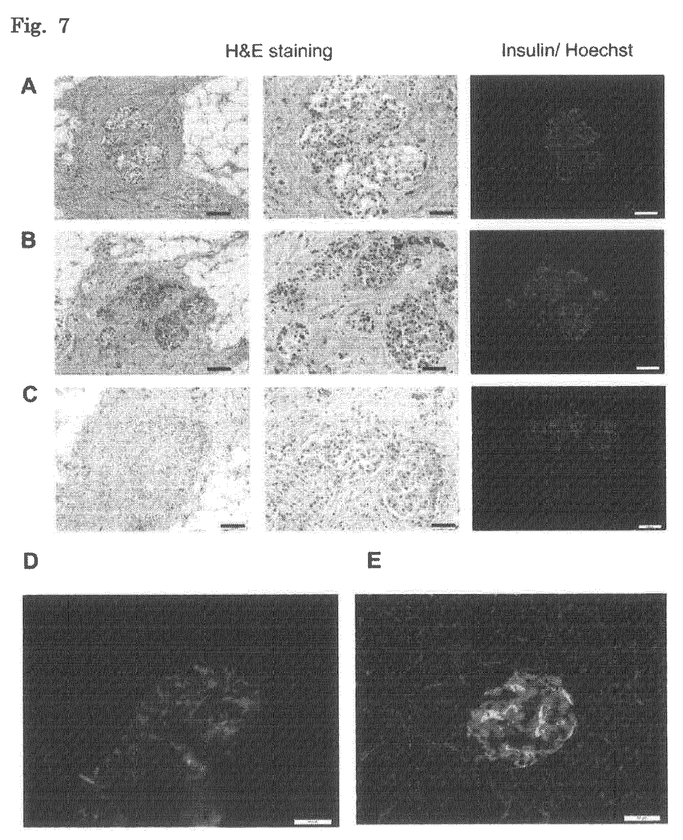

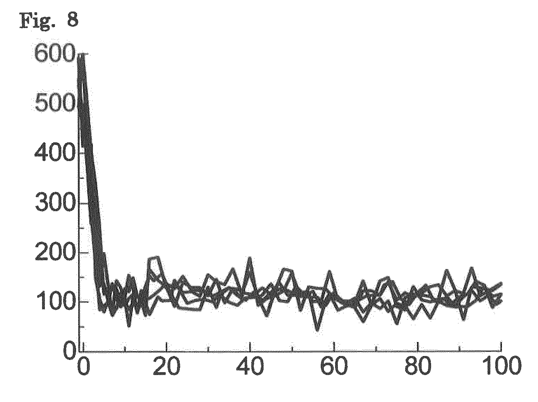

FIGs. 7 (A) to (E) illustrate the survival and revascularization of allogeneic pancreatic islets which were transplanted in angiogenesis-induced subcutaneous sites. FIGs. 7 (A) to (C) are stained images showing: (A) F344-ACI pancreatic islet grafts; (B) Lewis-ACI pancreatic islet grafts; and (C) F344-Wistar pancreatic islet grafts; which were transplanted into the angiogenesis-induced subcutaneous spaces and recovered 94 days, 102 days, and 130 days after the transplantation, and subjected to haematoxylin and eosin (H&E) staining and immunofluorescent staining for insulin and nuclei. Scale bars: 100 µm (images on the left and the right columns); 50 µm (middle columns). FIGs. 7 (D) and (E) are stained images showing: (D) pancreatic islet grafts in the subcutaneous site 30 days after allogeneic transplantation; and (E) pancreatic islets in the pancreas of the normal ACI rat; following in vivo perfusion with FITC-lectin for testing the blood vascular system. Scale bar: 50 µm. Four point five percent agarose gel rods each having a diameter of 4 mm and a length of 25 mm were freeze-dried. To each of the rods, 50 µg of fibroblast growth factor (bFGF) was added to be used for inducing the angiogenesis. Eight week-old, male ACI rats were used as recipients. A quantity of 60 mg/kg of streptozotocin (STZ) was administered to each of the ACI rats intraperitoneally to induce diabetes. After administering STZ two times in a row, those with blood glucose levels greater than 400 mg/dl were selected to be used as diabetic recipient rats. Three days after the administration of STZ, one of the agarose gel rods for inducing angiogenesis was transplanted subcutaneously to each of the left and right sides of the back of the respective ACI rats. The pancreatic islets were separated from the pancreases of F344 rats using a collagenase method. The F344 rats which are the donors of the pancreatic islets and the diabetic ACI rats are allogeneic to each other. One week after the implantation of the agarose gel rods, incisions were made on the skin of the rats to remove the agarose gel rods, and 1,500 pieces of pancreatic islets were transplanted to each of the two dorsal subcutaneous cavities formed. The agarose gel rods were able to be removed, without the adhesion of tissue thereto. After the transplantation, the blood glucose levels of the rats were measured once every two to three days. As shown in FIG. 8, the examination of five diabetic ACI rats revealed that the blood glucose levels were normalized in all five rats, over a period of 100 days or more. The normalization of the blood glucose levels suggests that the allogeneic pancreatic islets have survived in a state capable of secreting insulin, and that transplantation spaces where rejection is adequately suppressed have been formed.
FIG. 8 shows the changes in the blood glucose levels of the diabetic ACI rats, after the transplantation of pancreatic islets from the F344 rats to the dorsal subcutaneous sites of the ACI rats. It can be seen that the blood glucose levels were decreased due to the transplantation of allogeneic pancreatic islets, suggesting the survival of the transplanted pancreatic islets in a state capable of secreting insulin. The blood glucose levels were also normalized when agarose gel rods which do not contain heparin were used.

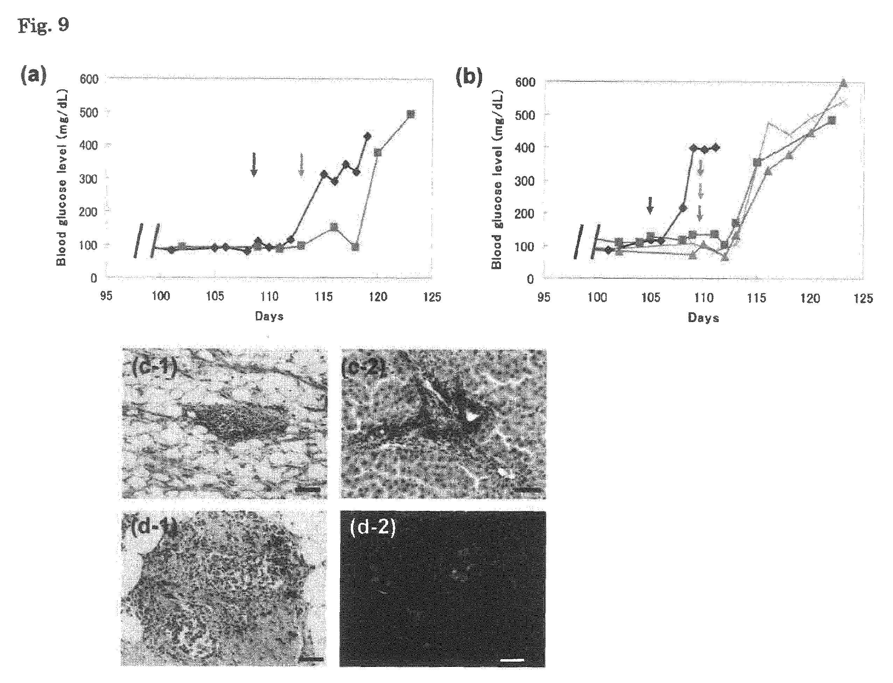

FIGs. 9 (A) to (D) illustrate the rejection of subcutaneously transplanted pancreatic islet grafts which have been successfully engrafted. The successfully engrafted pancreatic islets were obtained by: implanting agarose rods containing bFGF subcutaneously, removing the agarose rods one week after the implantation thereof, and by transplanting pancreatic islets in the spaces formed after the removal of the agarose rods. Each of all the recipient rats was transplanted with pancreatic islets, 1,500 pieces to each of the left and right dorsal subcutaneous sites of the respective recipients. The pancreatic islets transplanted on the left subcutaneous sites of the recipients were recovered on the day 91 to day 109 after the transplantation (n = 6). Even after the removal of the pancreatic islets from one of the two transplantation sites of the respective recipients, the normal blood glucose levels were maintained. FIG. 9 (A) shows the blood glucose levels (n = 2) in the diabetic ACI rats, which are ACI rats whose blood glucose levels had been normalized due to the transplantation of pancreatic islets, each of which further received the transplantation of 3,000 pieces of F344 pancreatic islets to the liver through the portal vein. Two arrows in the FIG. 9 (A) show the days on which the F344 pancreatic islets were transplanted to the livers of the rats. Approximately 10 days after the transplantation, the blood glucose levels in both two rats were increased, showing that the pancreatic islets in the subcutaneous tissue or in the livers were rejected. FIG. 9 (B): To each of the ACI rats whose blood glucose levels had been normalized due to the transplantation of the F344 pancreatic islet grafts, 10⁷ pieces of F344 splenocytes were transplanted intraperitoneally. Approximately five days after the transplantation, the blood glucose levels were increased (n = 4). This shows that pancreatic islets from F344 rats which had survived in the subcutaneous tissue were rejected. The arrows in FIG. 9 (B) show the days on which the F344 splenocytes were transplanted. FIGs. 9 (C-1) and (C-2) show haematoxylin and eosin (H&E) stained images of subcutaneous tissue (C-1) and liver tissue (C-2) containing allogeneic pancreatic islet grafts, which were recovered 14 days after the transplantation of the F344 pancreatic islets through the portal vein. No pancreatic islet tissue was observed, and the accumulation of white blood cells was confirmed. Scale bars: (C-1) 100 µm; (C-2) 50 µm. FIGs. 9 (D-1) and (D-2) show stained images of the tissue sections following the intraperitoneal injection of F344 splenocytes: stained with H&E (D-1); and immunofluorescently stained for insulin and nuclei (D-2). Scale bar: 50 µm. It can be seen from these histological images that, although a few insulin-positive cells are present, a large number of white blood cells are accumulated around the insulin-positive cells, suggesting that the pancreatic islets are being rejected.

### EMBODIMENTS FOR CARRYING OUT THE INVENTION

The device according to the present invention comprises a biocompatible structure containing an angiogenesis inducing factor, and it can be used by being implanted subcutaneously. When this device is implanted subcutaneously, a large number of microvessels will be formed around the device, due to the effect of the angiogenesis inducing factor which is released gradually from the biocompatible structure. It should be noted that, after making an incision on the skin for implanting the device subcutaneously, the site of the incision is sutured, and the device is kept implanted for a certain period of time. When sufficient amount of small-diameter blood vessels are formed, the skin is cut open again to remove the device, and a transplantation material is implanted into a transplantation space surrounded by small-diameter blood vessels. By implanting the device according to the present invention subcutaneously for one day or more, (for example, one to 25 days) in a mammal, the device is capable of forming a space where immunological rejection is suppressed to the extent to allow allogeneic cells or allogeneic micro-tissue transplanted therein to survive and function for a long period of time. Therefore, the angiogenesis inducing factor exhibits a sufficient effect, if it is retained in the device for about 12 hours. Examples of the mammal include a human, a monkey, a dog, a cat, a mouse, a rat, a rabbit, a cattle, a swine, a sheep, a rabbit, and the like. A human is preferred. By removing the device of the present invention from the subcutaneous site at which it was implanted, it is possible to form an immunological rejection-free transplantation space. When a transplantation material, such as cells (for example, allogeneic cells), micro-tissue (allogeneic micro-tissue), or the like which usually elicits rejection, is implanted to the transplantation space, surprisingly, an immune response is suppressed within the space under immunosuppressant-free conditions, thereby allowing the survival of the transplantation material. The transplantation material may be syngeneic or allogeneic.

The space, which is formed by subcutaneously implanting the device according to the present invention for a predetermined period of time to allow the formation of small-diameter blood vessels in the surrounding tissue, and then by removing the device, will be a space where immunological rejection is suppressed under immunosuppressant-free conditions. As used herein, the thus formed space is sometimes referred to as a "transplantation space", meaning that it is a space suitable for transplantation.

The transplantation space according to the present invention allows a transplantation material, such as pancreatic islets, to survive for a long period of time after the transplantation. Since the transplanted grafts which have been successfully engrafted in subcutaneous tissue are surrounded by small-diameter blood vessels, they can be supplied with sufficient oxygen and nutrients. Further, since a large number of white blood cells exist within the small-diameter blood vessels, it is assumed that these white blood cells derived from the small-diameter blood vessels will be supplied into the transplantation space. Unexpectedly, the number of the white blood cells which exist around the grafts is not so high, although slightly higher than the number of those existing in untreated tissue. It is considered that immunological rejection is suppressed due to the suppressor leukocytes and lymphocytes present in the tissue surrounding the transplantation space.

The transplantation material is preferably cells. In cases where a tissue is transplanted, a micro-tissue is preferred. Examples of the transplantation material include allogeneic cells and allogeneic micro-tissues, as well as syngeneic cells and syngeneic micro-tissues. When allogeneic cells or allogeneic micro-tissue are/is transplanted subcutaneously without taking particular measures to prevent rejection, usually, rejection occurs swiftly. However, when transplanted in the transplantation space according to the present invention, it is possible to suppress and to avoid the rejection. In other words, according to the present invention, a desirable transplantation site for both the allogeneic and syngeneic materials can be provided. As used herein, the "micro-tissue" refers to a tissue having a thickness of about 10 to 500 µm. The surface area thereof is not particularly limited, and the micro-tissue may have a large surface area. Basically, there are only a few blood vessels formed in the subcutaneous tissue. By implanting the device according to the present invention, the formation of microvessels will be induced in the tissue surrounding the device, but if the transplantation material is too large, sufficient oxygen or nutrients may not be supplied to the transplantation material. On the other hand, even if the transplantation material has a large surface area, it can be dealt with by increasing the surface area of the subcutaneous site into which the device according to the present invention is implanted.

The transplantation material is not particularly limited as long as it is capable of secreting a bioactive substance. Examples of the bioactive substance include hormones, cytokines, neurotransmitters, proteins (such as enzymes), and the like. Examples of hormones include insulin, glucagon, thyroxine (T4), triiodothyronine (T3), calcitonin, parathyroid hormone (PTH), cortisol, aldosterone, sex steroid hormones, catecholamine, androgen, estrogen, progesterone, growth hormone, prolactin, vasopressin, oxytocin, and the like. Examples of cytokines include: interleukins, interferons, colony stimulating factor (CSF), granulocyte colony stimulating factor (G-CSF), erythropoietin (EPO), epidermal growth factor (EGF), fibroblast growth factor (FGF), platelet-derived growth factor (PDGF), hepatocyte growth factor (HGF), transforming growth factor (TGF) tumor necrosis factor (TNF-α), lymphotoxin (TNF-β), leptin, nerve growth factor (NGF), and the like. Examples of proteins include albumin, heme protein, enzymes such as esterase, protease, transferase, dehydrogenase, oxidase, reductase, isomerase and synthase; and the like.

Examples of the transplantation material which is capable of secreting a bioactive substance as described above include: pancreatic islets, secreting cells in nervous system, thyroid cells, adrenal gland (cortex, medulla) cells, parathyroid cells, kidney cells, liver cells and micro-tissue including any of the above mentioned cells. Examples of the transplantation material further include any cells induced from embryonic stem cells and induced pluripotent stem cells. The transplantation material includes cells effective for treating a genetic disease which causes a condition where a specific gene product is not produced, or in which only a gene product having a reduced function is produced, due to gene defects or genetic mutation.

Examples of the angiogenesis inducing factor include: vascular endothelial growth factor (VEGF), basic fibroblast growth factor (bFGF), acidic fibroblast growth factor (aFGF), hepatocyte growth factor, platelet-derived growth factor (PDGF), transforming growth factor-β (TGF-β), placental growth factor, epidermal growth factor, Osteonectin, angiopoietin 1 (Ang1), angiopoietin 2 (Ang2) and the like. One kind of these factors can be used alone or two or more kinds may be used in combination. By further incorporating heparin or heparan sulfate into the biocompatible structure, it is possible to enhance the induction of the formation of blood vessels. The amount of the angiogenesis inducing factor to be incorporated into the structure varies depending on the type of the factor to be used, and it is not particularly limited. However, it is preferably an amount capable of inducing angiogenesis of 2 to 50 mg/g tissue, in terms of the amount of hemoglobin, more preferably, an amount capable of inducing angiogenesis of 3 to 40 mg/g tissue, in terms of the amount of hemoglobin, still more preferably an amount capable of inducing angiogenesis of 4 to 30 mg/g tissue, in terms of the amount of hemoglobin, and particularly preferably, an amount capable of inducing angiogenesis of 5 to 25 mg/g tissue. Further, although the amount of the angiogenesis inducing factor to be incorporated into the structure varies depending on the type of the factor to be used, and it is not particularly limited, it is preferably an amount which is capable of increasing the amount of hemoglobin, by implanting the device according the present invention in subcutaneous tissue for one week, five to 150 times the initial amount of hemoglobin before the implantation of the device into the subcutaneous tissue, in terms of the amount of hemoglobin; more preferably, it is an amount which is capable of increasing the amount of hemoglobin 10 to 100 times the initial amount, and still more preferably, an amount which is capable of increasing the amount of hemoglobin 15 to 70 times the initial amount. In addition, although the amount of the angiogenesis inducing factor to be incorporated into the structure varies depending on the type of the factor to be used, and it is not particularly limited, it is preferably an amount which is capable of increasing the amount of hemoglobin, by implanting the device according the present invention in subcutaneous tissue for one week, two to 15 times, in terms of the amount of hemoglobin, as compared to the amount of hemoglobin when a device which does not contain an angiogenesis inducing factor is implanted under the same conditions; more preferably, it is an amount which is capable of increasing the amount of hemoglobin two to 10 times, and still more preferably, an amount which is capable of increasing the amount of hemoglobin two to five times. In cases where basic fibroblast growth factor is used, for example, the amount thereof to be incorporated per device is usually about 20 µg/cm³ to 500 µg/cm³, preferably about 30 µg/cm³ to 300 µg/cm³, and more preferably about 40 µg/cm³ to 150 µg/cm³.

The biocompatible structure according to the present invention contains a hydrogel, a sponge, a porous polymer block and the like. Examples of the material constituting the hydrogel include: cellulosic derivatives such as hydroxypropyl methylcellulose (HPMC), sodium carboxymethylcellulose (CMC-Na), hydroxyethylcellulose (HEC); gelatin, alginic acid, albumin, collagen, fibrin, starch, agarose, agar-agar, dextran, pullulan, pectin, hyaluronic acid, chondroitin sulfate, heparin, chitin, and chitosan; chemically crosslinked products and crosslinked products obtained by radiation, of synthetic polymers such as: polyvinyl alcohol, polyvinylpyrrolidone, polyethylene glycol, poly-2-hydroxyethylmethacrylate, poly-2-hydroxyethylacrylate, polyacrylamide, polyisopropylacrylamide, polyacrylic acid, polymethacrylic acid, polysulfone, polyethylene, polypropylene, polycarbonate, polytetrafluoroethylene, and polyethylene terephthalate; crosslinked products of copolymers of monomers constituting the above mentioned polymers; polyion complex membranes of a polyanion and a polycation such as poly-L-lysine; and the like. The biocompatible structure as described above may also be produced by freeze-drying a gel-like product.

Examples of the sponge include porous media made of the above mentioned polymers, such as sponges of cellulose, agarose, chitosan, and collagen. Examples of the material constituting the porous medium include organic or inorganic materials, such as gelatin, silicone, and apatite. The biocompatible structure is preferably composed of a hydrogel, and particularly preferably composed of an agarose gel.

The biocompatible structure can be prepared, for example, in the form of a film, a sheet, a stick, a tube (such as a cylinder, an elliptic cylinder, and a rectangular cylinder). The biocompatible structure may be hollow, and contains an angiogenesis inducing factor on the surface or inside the biocompatible structure. The biocompatible structure may be in any form as long as the surface thereof is biocompatible, and allows for the body fluid to pass therethrough. The structure may have mesh-like or sponge-like porous cavities, which allow the infiltration of cells into the structure. Further, it may have cavities having a relatively large pore size which allow the infiltration of blood vessels into the structure. In cases where the induced microvessels infiltrate into the structure, a transplantation material may be introduced inside the biocompatible structure without removing the structure. In cases where the induced microvessels exist only on the surface of the structure, the structure may be removed, and the transplantation material may then be transplanted into the space formed by removing the structure.

The biocompatible structure of the present invention may include a support composed of, for example, a mesh made of a ceramic such as alumina or zirconia, or a metal such as stainless steel or titanium alloy, or a porous medium having continuous holes, with the outer surface of the support covered by a biocompatible material(s) constituting the hydrogel, sponge, or porous medium.

Examples of the method for allowing the biocompatible structure to carry the angiogenesis inducing factor include: a method in which the structure is simply impregnated with the angiogenesis inducing factor; a method in which a physicochemical interaction such as an electrostatic interaction or hydrophobic interaction is used to allow the structure to carry the angiogenesis inducing factor; a method in which a substance capable of biologically and specifically interacting with an angiogenesis inducing substance is incorporated into the structure, and the structure is allowed to carry the angiogenesis inducing factor utilizing the interaction therebetween; and a method in which, in cases where the structure is hollow, a solution of the angiogenesis inducing factor is introduced into the hollow portion. In cases where the structure is simply impregnated with the angiogenesis inducing factor, it is preferred that the density of the molecular net of the structure be adjusted so that the release rate of the angiogenesis inducing factor can be controlled. In cases where a physicochemical interaction such as an electrostatic interaction or hydrophobic interaction is used to allow the structure to carry the angiogenesis inducing factor, the physicochemical state of the angiogenesis inducing factor within the living body needs to be fully considered, before carrying out the procedure. For example, since a vascular endothelial growth factor or basic fibroblast growth factor is positively charged, as a whole molecule, within the living body, if the structure itself is negatively charged such as a structure made of acrylic acid or methacrylic acid, it is possible to allow the structure to carry the angiogenesis inducing factor utilizing the electrostatic interaction, and also to control the release rate of the factor. In cases where a biological specific interaction is utilized, it is preferred that a substance capable of improving the angiogenesis inducing factor and the activity thereof is incorporated into the structure in advance. For example, since a vascular endothelial growth factor or basic fibroblast growth factor interacts specifically with a mucopolysaccharide such as heparin or heparan sulfate, the incorporation of the angiogenesis inducing factor into the structure can be achieved by preparing a gel carrying a mucopolysaccharide such as heparin, and then by impregnating the gel with the growth factor. It is also possible to mix all of the above mentioned three components simultaneously to produce the structure of interest, depending on the material to be used in the production of the structure.

In order to allow the angiogenesis inducing factor to be released in a sustained release manner, various types of techniques related to DDS can be used.

The device according to the present invention is implanted subcutaneously. Specifically, the device is implanted subcutaneously by: making a small incision on the skin; forming a cavity in subcutaneous tissue in which only a few blood vessels exist, using a spatula or the like; implanting the device into the cavity; and then by suturing the site of incision. When the device is kept implanted in this state for a period of one to 35 days, preferably two to 28 days, more preferably three to 21 days, particularly preferably four to 14 days, blood vessels (small-diameter blood vessels) will be formed in the tissue surrounding the device. The formation of the blood vessels can be observed visually, or can be confirmed using a medical device, such as near-infrared imaging equipment, blood vessel imaging equipment, or the like.

By implanting the device according to the present invention for a predetermined period of time, and removing the device afterwards, it is possible to transplant cells or micro-tissue into the space formed by removing the device, and to achieve the suppression of immune response in the space to the extent to allow the transplanted cells or the micro-tissue to survive and function for a long period of time. In other words, the device according to the present invention is capable of forming a space, when pancreatic islets are transplanted thereinto, which allows for controlling the blood glucose level of the recipient within the normal range for a period of time, for example, of 30 days or more, 60 days or more, 90 days or more, 100 days or more, 110 days or more, or 120 days or more, after the transplantation. When a transplantation material is transplanted in such a space, the transplantation material is capable of functioning immediately after the transplantation. For example, if pancreatic islets are transplanted in the space, the pancreatic islets are capable of producing insulin immediately after the transplantation, and the blood glucose level of the recipient will be normalized on the day of, or the day following, the transplantation. If the transplantation material is too thick, sufficient nutrients may not be supplied thereto, and thus the transplantation material needs to have an appropriate thickness. However, the surface area of the transplantation material is not particularly limited, and it is possible to carry out the transplantation on a region having a large surface area. Further, the transplantation may be carried out at one site, or may be carried out at multiple sites. A portion or all of the transplanted cells or tissue can be easily removed, as required.

The space formed by the device according to the present invention can be used for the transplantation of various types of tissues. In particular, the space is suitable for the transplantation of a tissue which is capable of functioning regardless of the site at which it is transplanted. Examples of such a tissue include tissues which secrete bioactive substances such as hormones, cytokines, neurotransmitters, enzymes and the like. Specific examples thereof include hepatocytes, pancreatic islets, and cells derived from an adrenal gland, a parathyroid gland, and the like. Further, the space can be used for the transplantation of a tissue which is induced to differentiate from somatic stem cells, induced pluripotent stem cells, embryonic stem cells or the like.

The transplantation of tissue to the transplantation site formed by using the device according to the present invention is useful for treating various types of diseases. Particularly, the effect of reducing the symptoms and improving the quality of life provided by the transplantation are greater in insulin dependent diabetes patients whose ability to produce insulin is absolutely insufficient and who are in need for administration of insulin externally by an injection or the like, as compared to the effect provided to the patients with other endocrine diseases. Therefore, the transplantation site-forming agent according to the present invention is particularly useful when used in the formation of a transplantation site for pancreatic islets in order to treat insulin dependent diabetes. One of the primary examples of the diabetes as described above is type I diabetes, but the present invention is also useful for the treatment of type II diabetes. Specifically, when type II diabetes is progressed, the volume of pancreatic β cells is reduced, and in the current situation, it is treated by administration of insulin. However, the use of the transplantation site-forming agent according to the present invention allows for treating such conditions. Further, in view of the fact that type II diabetes is developed due to the insulin resistance of the body being increased to exceed the insulin-producing ability in the pancreas, it is also effective to use the transplantation as a prophylactic treatment, before the administration of insulin becomes essential.

Further, the device according to the present invention is capable of forming a transplantation site where rejection can be suppressed even in cases where a tissue from another person is transplanted. Since, a tissue provided by another person is used in pancreatic islet transplantation for treating the insulin dependent diabetes, for example, a continuous administration of an immunosuppressant is usually required after the transplantation. However, by using the transplantation site-forming agent according to the present invention, it is possible to carry out pancreatic islet transplantation with less or no administration of an immunosuppressant. The primary disease in patients with insulin dependent diabetes is type I diabetes, and the cause for developing the type I diabetes is an autoimmune response. If it is possible to form a transplantation site where the suppression of immune response can be achieved, the protection of the tissue transplanted therein from the autoimmune response can be expected, and thus, the present method is highly useful also in terms of preserving the function of the transplanted pancreatic islets for a long period of time.

### EXAMPLES

The present invention will now be described in detail by referring to Examples and Comparative Examples. However, the present invention is in no way limited by the following Examples.

In Examples, (1) immunostaining, (2) in vivo perfusion with FITC-lectin, and (3) statistical analysis, were carried out as follows.

### (1) Tissue staining

Collected tissues containing pancreatic islet grafts were fixed in a 4% paraformaldehyde solution in phosphate buffered saline (PBS), and thin tissue sections (thickness: 4 µm) were prepared for the subsequent operation. Insulin, CD4 T cells and CD8 T cells were immunostained. Haematoxylin and eosin (H&E) staining and immunofluorescent staining for macrophages and granulocytes were carried out (Luan N M, Iwata H. Biomaterials 2013; 34: 5019-5024.). For immunostaining, spleen tissues and samples without primary antibodies were used as positive controls and negative controls, respectively. An antibody against CD4 T cells (rabbit polyclonal antibody; Novus Biologicals, Littleton, CO), an antibody against CD8 T cells (mouse monoclonal antibody, OX-8; Novus Biologicals), an antibody against macrophages (CD68) (mouse monoclonal antibody; ED1, Novus Biologicals) and an antibody against granulocytes (mouse monoclonal antibody; HIS48, Novus Biologicals) were purchased. The thin tissue sections were treated with Blocking One solution (Nacalai Tesque) for one hour to block nonspecific adsorption of antibodies, followed by incubation with a primary antibody against CD4 T cells (1:50, in the Blocking One solution), a primary antibody against CD8 T cells (1:50, in the Blocking One solution), a primary antibody against macrophages (1:50, in the Blocking One solution) and a primary antibody against granulocytes (1:50, in the Blocking One solution), overnight at 4°C. After washing the resultants with PBS containing 0.05% Tween-20, the sections were treated with Alexa Fluor 594 anti-rabbit IgG antibody or Alexa Fluor 488 anti-mouse IgG antibody (1:200, in the Blocking One solution; Invitrogen, Life Technologies, Carlsbad, CA) for one hour at room temperature, followed by washing with PBS containing 0.05% Tween-20. The nuclei were counterstained with Hoechst 33258 (Dojindo, Kumamoto, Japan).

### (2) In vivo perfusion with fluorescein isothiocyanate (FITC)-lectin for measuring blood vascular system

To ACI rats in which agarose rods were implanted for one week to induce angiogenesis, or ACI recipient rats 30 days after the transplantation of F344 pancreatic islets, 1 mL of a physiological saline containing 200 µg of FITC-lectin (Vector Labs, Burlingame, CA) and 1,000 IU of heparin were injected intravenously. After 15 minutes of circulation, subcutaneous tissues and pancreases were swiftly excised from the rats. Then, the subcutaneous tissues and pancreases were briefly washed twice with a physiological saline solution, fixed, and snap-frozen in liquid nitrogen. Thin tissue sections (thickness: 4 µm) were prepared, and immunofluorescent staining for insulin was carried out according to the usual manner.

### (3) Statistical analysis

Two groups were compared using the Student t-test. p < 0.05 was considered significant. All the statistical calculations were carried out using JMP ver. 5.1.1 (http://www. jmp.com/).

### Example 1

Four point five percent agarose gel rods each having a diameter of 4 mm and a length of 25 mm were freeze-dried. To each of the rods, 50 µg of fibroblast growth factor (bFGF) and 25 µg of heparin were added to be used as the device for inducing angiogenesis. Eight week-old, male ACI rats were used as recipients. A quantity of 60 mg/kg of streptozotocin (STZ) was administered to each of the ACI rats intraperitoneally to induce diabetes. After administering STZ two times in a row, those with blood glucose levels greater than 400 mg/dl were selected to be used as diabetic recipient rats. Three days after the administration of STZ, one of the agarose gel rods for inducing angiogenesis was transplanted subcutaneously to each of the left and right sides of the back of the respective ACI rats. The pancreatic islets were separated from the pancreases of F344 rats using a collagenase method. F344 rats, which are the donors of the pancreatic islets, and the diabetic ACI rats are allogeneic to each other. One week after the implantation of the devices, incisions were made on the skin of the rats to remove the devices, and 1,500 pieces of pancreatic islets were transplanted to each of the two dorsal subcutaneous cavities formed. The devices were able to be removed, without the adhesion of tissue thereto. After the transplantation, the blood glucose levels of the rats were measured once every two to three days. As shown in FIG. 1, the examination of 10 diabetic ACI rats revealed that the blood glucose levels were normalized in nine out of 10 rats, over a period of 100 days or more. The normalization of the blood glucose levels suggests that the allogeneic pancreatic islets have survived in a state capable of secreting insulin, and that transplantation spaces in which rejection is adequately suppressed have been formed.

FIG. 2 shows histological images of tissue sections. FIG. 2 (a) is a histological image of a tissue section obtained from the dorsal implantation site of the rat seven days after the subcutaneous implantation of the agarose rod containing 50 µg of fibroblast growth factor and 25 µg of heparin. It can be seen that a large number of microvessels have been formed. FIGs. (b-1) to (b-3) are histological images of tissue sections obtained from the transplantation site of the ACI rat whose blood glucose level has been normalized. FIG. 2 (b-1) is a haematoxylin and eosin stained image of a tissue section from the pancreatic islet transplantation site. The presence of a large number of cell conglomerates which are considered to be pancreatic islets, and the formation of a large number of microvessels passing through the cell conglomerates are observed. This shows that the allogeneic pancreatic islets derived from the F344 rats and transplanted in the transplantation space formed by implantation of the device, were not rejected, and that the pancreatic islets have been supplied with oxygen and nutrients through the microvessels, and have successfully been engrafted in the transplantation space in a state capable of surviving for a long period of time. FIG. 2 (b-2) is a stained image of a tissue section from an approximately the same site as the tissue section shown in (b-1), immunostained for insulin. The portion of the tissue stained green is the portion in which cells secreting insulin are present. When the tissue section immunofluorescently stained for insulin was examined using a fluorescence microscope, the presence of insulin positive cells were observed at the position corresponding to the position at which the cell conglomerates assumed to be the pancreatic islets were observed in the tissue section stained with haematoxylin and eosin. This proves that the transplanted pancreatic islets have maintained the insulin-producing function. FIG. 2 (b-3) is an enlarged image of the portion of the image shown in (b-1) in which transplanted pancreatic islets exist. The presence of the transplanted pancreatic islets and the absence of inflammatory cells were confirmed. The absence of inflammatory cells proves that, by implanting the device according to the present invention subcutaneously, it is possible to form a space (transplantation space) where the suppression of immune system is achieved.

Next, the intraperitoneal glucose tolerance test was performed for normal ACI rats and STZ-ACI rats transplanted with F344 pancreatic islets or Lewis pancreatic islets, one to three months before the test. The results are shown in FIG. 6A. Areas under the curves for normal ACT rats, STZ-ACI rats transplanted with 3,000 pieces of F344 pancreatic islets, and STZ-ACI rats transplanted with 3,000 pieces of Lewis pancreatic islets, were 19867 ± 1897, 22835 ± 2023 and 20180 ± 1286 mg min/dL, respectively. Further, the results of the measurement of the plasma insulin levels are shown in FIG. 6B. The plasma insulin levels of the STZ-ACI rats (IV) each transplanted with 3,000 pieces of F344 pancreatic islets into the angiogenesis-induced subcutaneous spaces, or of the STZ-ACI rats (III) each transplanted with 3,000 pieces of Lewis pancreatic islets into the angiogenesis-induced subcutaneous spaces, were measured 40 days and 90 days after the pancreatic islet transplantation, and the measured values were 1.56 ± 0.23 ng/mL (IV) and 1.73 ± 0.140 ng/mL (III), respectively. The plasma insulin levels of the normal ACI rats (I) and diabetic ACI rats (II) were 1.39 ± 0.32 ng/mL and 0.31 ± 0.06 ng/mL, respectively. The plasma insulin levels of the recipient rats were sufficiently maintained by the pancreatic islet grafts (FIG. 6 B).

### Comparative Example 1

In the same manner as in Example 1, eight week-old, male ACI rats were used as recipients. A quantity of 60 mg/kg of streptozotocin (STZ) was administered to each of the ACI rats intraperitoneally to induce diabetes. After administering STZ two times in a row, those with blood glucose levels greater than 400 mg/dl were selected to be used as diabetic recipient rats. The pancreatic islets were separated from the pancreases of F344 rats using a collagenase method. To the recipient rats in which angiogenesis had not been introduced, 1,500 pieces of allogeneic pancreatic islets were transplanted subcutaneously to each of the transplantation sites at both sides of the back of the rats. After the transplantation, the blood glucose levels of the rats were measured once every two to three days. The examination of four diabetic ACI rats revealed that the blood glucose level of none of the rats was normalized. This is considered as follows. Since there are extremely few blood vessels in the subcutaneous tissue of the rats, the concentration of oxygen is low in the tissue, which probably caused an insufficient oxygen supply to the transplanted pancreatic islets, resulting in death of pancreatic islets due to lack of oxygen.

### Comparative Example 2

In the same manner as in Example 1, eight week-old, male ACI rats were used as recipients. A quantity of 60 mg/kg of streptozotocin (STZ) was administered to each of the ACI rats intraperitoneally to induce diabetes. After administering STZ two times in a row, those with blood glucose levels greater than 400 mg/dl were selected to be used as diabetic recipient rats. The pancreatic islets were separated from the pancreases of F344 rats using a collagenase method. Three thousand pieces of the separated pancreatic islets were transplanted into the liver through the portal vein. After the transplantation, the blood glucose levels of the rats were measured once every two to three days. The examination of three diabetic ACI rats revealed that, as shown in FIG. 3, although the blood glucose levels of all three rats were normalized temporarily, the blood glucose levels reverted to the high preoperative levels, within 10 days. It has been confirmed that, when allogeneic pancreatic islets are transplanted into the liver through the portal vein, the pancreatic islets initially exist in a state capable of producing insulin, but they will be rejected within 10 days, thereby losing their functions.

### Example 2

Four point five percent agarose gel rods each having a diameter of 4 mm and a length of 25 mm were freeze-dried. To each of the rods, 50 µg of fibroblast growth factor (bFGF) and 25 µg of heparin were added to be used for induction of angiogenesis. Eight week-old, male ACI rats were used as recipients. A quantity of 60 mg/kg of streptozotocin (STZ) was administered to each of the ACI rats intraperitoneally to induce diabetes. After administering STZ two times in a row, those with blood glucose levels greater than 400 mg/dl were selected to be used as diabetic recipient rats. Three days after the administration of STZ, one of the agarose gel rods for inducing angiogenesis was transplanted subcutaneously to each of the left and right sides of the back of the respective ACI rats. The pancreatic islets were separated from the pancreases of Lewis rats using a collagenase method. One week after the implantation of the devices, incisions were made on the skin of the rats to remove the devices, and 1,500 pieces of pancreatic islets were transplanted to each of the two dorsal subcutaneous cavities (transplantation spaces) formed. After the transplantation, the blood glucose levels of the rats were measured once every two to three days. On the days indicated with arrows, as shown in FIG. 4, the skins of the both sides of the back of the rats were removed along with the transplanted pancreatic islets. As shown in FIG. 4, the examination of four diabetic ACI rats revealed that the blood glucose levels of all four rats had been normalized until the day on which the transplanted pancreatic islets were removed from their backs, and the blood glucose levels of these rats reverted to the high preoperative levels, as soon as the pancreatic islets were removed. This results shows that the transplanted pancreatic islets had been secreting the insulin to normalize the blood glucose level.

The summarized results of the allogeneic transplantation between rats are shown in the following Table 1.

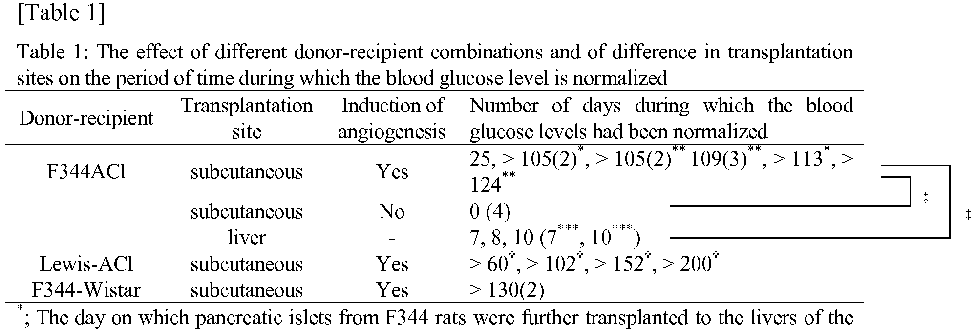

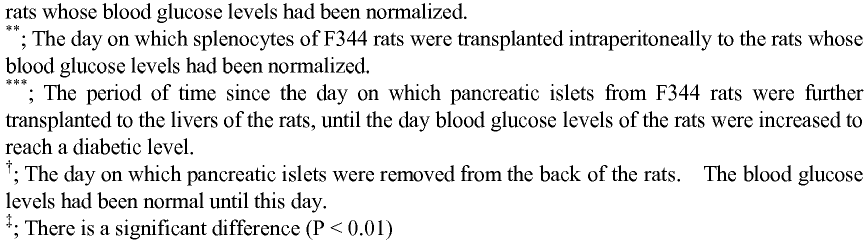

### Example 3

Four point five percent agarose gel rods each having a diameter of 4 mm and a length of 20 mm were freeze-dried. To each of the rods, 50 µg of fibroblast growth factor (bFGF) and 25 µg of heparin were added to be used for induction of angiogenesis. Six week-old, male BALB/c mice were used as recipients. A quantity of 200 mg/kg of streptozotocin (STZ) was administered to each of the BALB/c mice intraperitoneally to induce diabetes. After administering STZ two times in a row, those with blood glucose levels greater than 400 mg/dl were selected to be used as diabetic recipient mice. Three days after the administration of STZ, one of the agarose gel rods for inducing angiogenesis was transplanted subcutaneously to each of the left and right sides of the back of the respective BALB/c mice. The pancreatic islets were separated from the pancreases of C57BL/6 mice using a collagenase method. One week after the implantation of the devices, incisions were made on the skin of the rats to remove the devices, and 250 pieces of islets of Langerhans (pancreatic islets) were transplanted to each of the two dorsal subcutaneous cavities formed. After the transplantation, the blood glucose levels of the rats were measured once every two to three days. The examination of eight diabetic BALB/c mice revealed that the blood glucose levels were normalized in five mice for over 30 days or more.

### [Examples 4 to 5]

Examples 4 to 5 and Comparative Examples 3 to 4 were carried out under the same conditions as in Example 1, except that the amounts of the fibroblast growth factor (bFGF) added were changed to the amounts shown in Table 2, respectively. The amount of microvessels induced in the subcutaneous tissue and the effect of normalizing the blood glucose level were examined for each of the rats in the Examples and Comparative Examples.

The results are also shown in Table 2. The amount of the microvessels induced in the subcutaneous tissue was evaluated in terms of the amount of hemoglobin contained in 1 g of tissue (mg/g tissue).

In vivo perfusion with FITC-lectin was carried out for rats in Example 4, which were diabetic rats transplanted with devices each containing bFGF (50 µg) and heparin for one week, in the dorsal subcutaneous sites, and the blood vascular system in the angiogenesis-induced subcutaneous sites was investigated, as shown in FIG. 5. A large number of lectin-positive blood vessels were observed in the subcutaneous site, which is indicative of a dense blood vessel distribution formed at this site. This allows us to expect that the survived pancreatic islet grafts have been supplied with sufficient nutrients and oxygen.

Since blood vessels contain hemoglobin, the content of hemoglobin in the subcutaneous tissue was used for comparing the induction of angiogenesis semi-quantitatively (Kawakami Y, Iwata H, Gu Y J, et al., Pancreas 2001; 23: 375-381.). The hemoglobin content of the untreated subcutaneous tissue was 0.35 ± 0.55 mg/g tissue. The hemoglobin content of the tissue varies depending on the amount of bFGF introduced into the agarose rod to be implanted therein. The hemoglobin content of the tissue when an agarose rod containing 10 µg of bFGF was implanted therein was 3.30 ± 1.35 mg/g, which has no significant difference compared to the tissue untreated with the bFGF. When the bFGF content is increased to 50 µg/ rod, the hemoglobin content in the tissue is significantly increased to 9.40 ± 2.84 mg/g. An increased amount of bFGF, or a sustained release over a longer period of time, has a possibility of increasing the hemoglobin content.

Further, from the rats in Example 5 carrying long-term surviving pancreatic islet grafts, the pancreatic islet grafts transplanted subcutaneously on the left sides of the rats were retrieved during the period from the day 91 to the day 130, for histological measurement. Tissue sections were obtained from the rats whose blood glucose levels have been maintained within a normal range even after the transplanted grafts were removed from one of the two transplantation sites of the respective rats, and the sections were stained with H&E and Alexa 488-labeled anti-insulin antibody (FIGs. 7A, B, and C). In all three donor-recipient combinations, the pancreatic islets were clearly observed in the H&E stained sections, and the insulin-positive cells were clearly observed in the immunofluorescently stained sections. A large number of small blood vessels were observed in the pancreatic islet grafts, but the infiltration of lymphocytes was scarcely confirmed. The present inventors have also evaluated the formation of blood vessels in the subcutaneously transplanted pancreatic islet grafts by in vivo perfusion using FITC-lectin. As shown by FITC-lectin-stained cells around the insulin cells, small-diameter blood vessels were clearly detected in the pancreatic islet grafts (FIG. 7D). FIG. 7E is a stained image of a normal pancreatic tissue. The form of the pancreatic islets and the blood vessel distribution shown in FIGs. 7D and 7E are similar to each other. These data suggest that, the allogeneic pancreatic islets transplanted to the angiogenesis-induced subcutaneous sites have established a network of blood vessels which are connected to the blood vascular system of the host.

**[Table 2]**

| | Amount of bFGF added (µg) | Amount of hemoglobin (mg/g tissue) | The Effect of normalizing the blood glucose level |
|---|---|---|---|
| Example 4 | 10 | *3.30* ± *1.35mg*/*g* | The blood glucose levels were normalized in three out of 10 rats. |
| Example 5 | 50 | *9.40* ±*2.84mg*/*g* | Te blood glucose levels were normalized in nine out of 10 rats for 100 days or more. |
| Comparative Example 3 | Untreated | *0.55* ± *0.35mg*/*g* | No successful survival of the transplanted grafts in all four recipients. |
| Comparative Example 4 | 0 | *2.90* ± *0*.*75mg*/*g* | No successful long-term survival of the transplanted grafts in all five recipients. |

### Example 6

Four point five percent agarose gel rods each having a diameter of 4 mm and a length of 25 mm were freeze-dried. To each of the rods, 50 µg of fibroblast growth factor (bFGF) was added to be used for induction of angiogenesis. Eight week-old, male ACI rats were used as recipients. A quantity of 60 mg/kg of streptozotocin (STZ) was administered to each of the ACI rats intraperitoneally to induce diabetes. After administering STZ two times in a row, those with blood glucose levels greater than 400 mg/dl were selected to be used as diabetic recipient rats. Three days after the administration of STZ, one of the agarose gel rods for inducing angiogenesis was transplanted subcutaneously to each of the left and right sides of the back of the respective ACI rats. The pancreatic islets were separated from the pancreases of F344 rats using a collagenase method. The F344 rats, which are the donors of the pancreatic islets, and the diabetic ACI rats are allogeneic to each other. One week after the implantation of the devices (agarose gel rods), incisions were made on the skin of the rats to remove the devices, and 1,500 pieces of pancreatic islets were transplanted to each of the two dorsal subcutaneous cavities formed. The devices were able to be removed, without the adhesion of tissue thereto. After the transplantation, the blood glucose levels of the rats were measured once every two to three days. As shown in FIG. 8, the examination of five diabetic ACI rats revealed that the blood glucose levels were normalized in all five rats, over a period of 100 days or more. The normalization of the blood glucose levels suggests that the allogeneic pancreatic islets have survived in a state capable of secreting insulin, and that transplantation spaces in which rejection is adequately suppressed have been formed.

### Example 7

To the liver of each of the STZ-ACI recipients whose blood glucose levels had been normalized for 100 days or more due to the transplantation of F344 pancreatic islets obtained in the same manner as in Example 1, 3,000 pieces of F344 pancreatic islets were further transplanted through the portal vein. The blood glucose levels of those recipients were increased to 350 mg/dL within seven to ten days after the injection of the pancreatic islets (n = 2, FIG. 9A). The pancreatic islets in the livers and in the subcutaneous tissue both lost their insulin-producing functions. It can be seen from the tissue sections which were recovered 14 days after the injection of the pancreatic islets, that a large number of lymphocytes infiltrated to the region around the pancreatic islets in the liver and subcutaneous tissue, and that the pancreatic islets suffered a serious damage (FIGs. 9C-1 and 9C-2). Further, to each of the STZ-ACI recipients whose blood glucose levels had been normalized for 100 days or more due to the transplantation of F344 pancreatic islets obtained in the same manner as in Example 1, F344 splenocytes were administered intraperitoneally. The blood glucose levels of the STZ-ACI recipients were increased to exceed 350 mg/dL within four to seven days after the administration of F344 splenocytes (n = 4, FIG. 9B). It can be seen from the tissue sections which were recovered four days after the splenocyte injection that a large number of lymphocytes infiltrated to the region around the pancreatic islets, and that the number of the insulin-positive cells was decreased (FIGs. 9D-1 and 9D-2).

The results of Example 7 suggest that the immune response elicited by the cells of the rat of the same strain as the pancreatic islet donor, disrupts engraftment and has a fatal impact on the survival of the pancreatic islet grafts. At the same time, it is also suggested that an immunosuppressed site suitable for the transplantation and the survival of the allogeneic cells have been formed by the method using the device according to the present invention.

Further, it was confirmed that the method of the present invention allows the pancreatic islet grafts transplanted without the administration of an immunosuppressant to survive stably for a long period of time and to keep secreting insulin until a normal blood glucose level is achieved. These pancreatic islet grafts are rejected, if an allogeneic transplantation material which elicits immunological rejection is further transplanted to dramatically activate the immune system of the host. However, the transplanted pancreatic islets have survived in the same state as they survive in the pancreas, unless a particular operation as described above is carried out, and it is clear that the present method is extremely effective for treating diabetes.

### Industrial applicability

The transplantation of pancreatic islets obtained from deceased donors to diabetes patients is an excellent method for treating insulin dependent diabetes. However, since the number of deceased donors available is limited, this method is not expected to be established as a general treatment method. On the other hand, recent studies have reported that, it is possible to normalize the blood glucose level in diabetic model animals, by inducing differentiation of insulin-secreting cells, or even pseudo pancreatic islets, from induced pluripotent stem cells (iPS cells or the like) or embryonic stem cells (ES cells); encapsulating these cells or pancreatic islets in bags made of a semipermeable membrane to prevent them from being rejected, and then by transplanting them to these animals. If the induced differentiation of insulin-secreting cells, or even pseudo-pancreatic islets, from induced pluripotent stem cells (iPS cells or the like) or embryonic stem cells (ES cells) becomes easily feasible, it will be possible to treat diabetes by cell transplantation, without requiring the administration of an immunosuppressant or the use of bags made of semipermeable membrane, by carrying out the transplantation according to the method of the present invention. The method according to the present invention is an extremely potent method for providing an ideal treatment for diabetes. The fact that transplantation materials other than pancreatic islets can also be transplanted without requiring the administration of an immunosuppressant provides a great advantage.

## Claims

1. A device to be implanted subcutaneously for forming a transplantation space where rejection does not occur, wherein the device comprises a biocompatible structure containing an angiogenesis inducing factor.

2. The device according to claim 1, wherein a material to be transplanted in the transplantation space is allogeneic cells or allogeneic micro-tissue.

3. The device according to claim 2, wherein the material to be transplanted in the transplantation space is islets of Langerhans (pancreatic islets).

4. The device according to claim 2, wherein the material to be transplanted in the transplantation space is cells which have been induced to differentiate from somatic stem cells, embryonic stem cells or induced pluripotent stem cells.

5. The device according to claim 4, wherein the cells which have been induced to differentiate are insulin-secreting cells.

6. The device according to any one of claims 1 to 5, wherein the angiogenesis inducing factor is at least one selected from the group consisting of fibroblast growth factor, vascular endothelial growth factor, hepatocyte growth factor, platelet-derived growth factor, placental growth factor and epidermal growth factor.

7. The device according to any one of claims 1 to 6, wherein the biocompatible structure is selected from the group consisting of a hydrogel, a sponge and a porous medium.

8. The device according to any one of claims 1 to 7, further comprising heparin or heparan sulfate.

9. The device according to claim 2, wherein the material to be transplanted in the transplantation space is allogeneic cells or allogeneic micro-tissue.

10. The device according to any one of claims 1 to 9, wherein the device comprises the angiogenesis inducing factor in an amount capable of inducing angiogenesis of 2 mg to 50 mg per gram tissue, in terms of the amount of hemoglobin.

11. A method for forming a subcutaneous space where immunological rejection is suppressed under immunosuppressant-free conditions, in a mammal, the method comprising the steps of:
implanting the device according to any one of claims 1 to 10 subcutaneously in the mammal to allow the formation of small-diameter blood vessels around the device; and
removing the device.

12. A method for transplanting a transplantation material in a mammal under immunosuppressant-free conditions, the method comprising the steps of:
implanting the device according to any one of claims 1 to 10 subcutaneously in the mammal to allow the formation of small-diameter blood vessels around the device;
removing the device to form a space where immunological rejection is suppressed under immunosuppressant-free conditions; and
transplanting in the space a transplantation material selected from the group consisting of allogeneic cells and allogeneic micro-tissues.

13. The method according to claim 12, wherein the transplantation material is allogeneic cells or allogeneic micro-tissue.

14. A use of a device for allowing the survival of a transplantation material which has a possibility of being rejected immunologically, in subcutaneous tissue under immunosuppressant-free conditions,
wherein the device comprises a biocompatible structure containing an angiogenesis inducing factor, and
wherein the device is implanted in the subcutaneous tissue to allow the formation of blood vessels around the device, then the device is removed from the subcutaneous tissue to form a transplantation space where rejection does not occur, and the transplantation material which has a possibility of being rejected immunologically is transplanted in the transplantation space, to allow the survival of the transplantation material in the subcutaneous tissue under immunosuppressant-free conditions.

15. The use according to claim 14, wherein the material to be transplanted in the transplantation space is allogeneic cells or allogeneic micro-tissue.

16. A method for treating a disease, comprising the steps of:
implanting the device according to any one of claims 1 to 10 subcutaneously in a mammal to allow the formation of small-diameter blood vessels around the device;
removing the device to form a space where immunological rejection is suppressed under immunosuppressant-free conditions; and
transplanting a transplantation material for treating the disease in the space; wherein the transplantation material is selected from the group consisting of allogeneic cells and allogeneic micro-tissues.

17. The method for treating a disease according to claim 16, wherein the disease is diabetes, and the transplantation material is pancreatic islets.
